# FASCICULE DE BREVET EUROPEEN

(11) **EP 4 157 041 B1**
(45) Date de publication et mention de la délivrance du brevet: **08.05.2024**
(21) Numéro de dépôt: 21732599.2
(22) Date de dépôt: 30.04.2021
(51) Int. Cl.: A47C 20/04, A61G 1/00, A61B 5/00, A47C 21/00, A61G 7/015, A61G 7/018, A47C 31/00, A61B 5/024

(54) **LITS INTELLIGENTS MULTIFONCTIONNELS ET LEURS MODES DE FONCTIONNEMENT**
INTELLIGENTE MULTIFUNKTIONSBETTEN UND BETRIEBSMODI DAFÜR
MULTIFUNCTIONAL SMART BEDS AND OPERATING MODES THEREOF

(30) Priorité: 29.05.2020 US 202016888378; 23.04.2021 MA 53066
(43) Date de publication de la demande: 05.04.2023
(73) Titulaire: Saghiri, Khalid, Meknes (MA)
(72) Inventeur: Saghiri, Khalid, Meknes (MA)
(74) Mandataire: Bringer IP
(86) Numéro de dépôt international: PCT/MA2021/000005
(87) Numéro de publication internationale: WO 2021/242087

(56) Documents cités:
- WO-A1-2017/190085
- CN-A- 105 877 713
- KR-B1- 102 059 065
- US-A1- 2011 295 083
- US-A1- 2015 334 482
- US-A1- 2017 135 881
- US-A1- 2019 209 082
- US-A1- 2020 060 907
- US-B2- 7 869 903
- US-B2- 9 011 346

## Description

### CONTEXTE

Les gens passent environ le tiers de leur vie au lit. De nombreuses études ont montré que la qualité et la durée du sommeil ont un effet majeur sur la concentration, la santé mentale / physique et le bien-être émotionnel. De plus, les lits ont d'autres utilisations que le sommeil, comme la récupération de la santé, l'intimité et le repos général (par exemple, lire, regarder la télévision). Enfin, le même lit peut être partagé par plusieurs personnes (p. Ex., Conjoints, enfants) ayant des habitudes de sommeil et des exigences de lit différentes (p. Ex. fermeté du matelas).

Cependant, les lits modernes sont à la traîne des progrès dans d'autres domaines technologiques, tels que les smartphones et les maisons intelligentes. Les lits conventionnels sont encore généralement limités aux matelas plats. Dans des cas limités, un réglage manuel de la fermeté du matelas est disponible. Les lits spécialisés (par exemple, les lits d'hôpitaux) permettent également un réglage manuel de leurs sections de tête (par exemple, en changeant l'angle d'inclinaison). Cependant, la plupart de ces lits sont généralement limités à des commandes manuelles et offrent des fonctionnalités très limitées, axées sur des utilisations particulières de ces lits. Les documents d'art antérieur pertinents sont US2020/060907A1, US7869903B2, CN105877713A, KR102059065B1, US2017/135881, US2015/334482A1, US9011346B2, US2019/209082 A1 et US2011/295083A1.

Ce qui est nécessaire, c'est un lit intelligent multifonctionnel, configuré pour analyser de multiples caractéristiques liées à l'utilisateur et à son environnement et pour fournir diverses options opérationnelles et de rapports basés sur l'analyse complète de ces caractéristiques.

### RESUME

On décrit ici des lits intelligents multifonctionnels et des procédés de fonctionnement de ceux-ci. Plus précisément, un lit intelligent multifonctionnel comprend un contrôleur, configuré pour recevoir une entrée de divers capteurs (par exemple, des capteurs de force positionnées dans des pieds de lit, des thermomètres et / ou des microphones) et / ou un utilisateur. Ces entrées sont analysées et utilisées pour contrôler divers composants du lit pour améliorer la qualité du sommeil, pour surveiller la santé des utilisateurs, évaluer la relation de couple, etc. Par exemple, ces entrées peuvent être utilisées pour contrôler la fermeté de différentes parties du matelas, changer les orientations des sections tête, torse et / ou jambes, ajuster la lumière, etc. Dans certains exemples, le lit intelligent génère un rapport indiquant une ou plusieurs durées de sommeil, le profil de profondeur de sommeil, l'étendue du sommeil, la fréquence cardiaque, la fréquence respiratoire, les conditions environnementales, la relation de couple, etc. En outre, dans certains exemples, un lit intelligent multifonctionnel est également configuré pour contrôler des appareils externes dans le même environnement, par exemple des interrupteurs intelligents, des thermostats intelligents.

Selon l'invention, un lit intelligent multifonctionnel comprend un cadre, une pluralité de pieds, chacun des pieds de lit comprenant un capteur de force et couplé au cadre, un matelas, positionné sur et supporté par un cadre, une pluralité de capteurs environnementaux, un contrôleur, couplé en communication aux capteurs de force dans chacun de la pluralité des pieds et à chacun de la pluralité de capteurs environnementaux, et une pluralité d'actionneurs, couplé en communication au contrôleur et configuré pour changer au moins une ou plusieurs caractéristiques du lit intelligent multifonctionnel basé sur l'entrée reçue par le contrôleur. Par exemple, la ou les caractéristiques du lit comprennent au moins une parmi les suivantes : la fermeté du matelas et une ou plusieurs positions des différentes sections du cadre l'une par rapport à l'autre.

Selon l'invention, la pluralité d'actionneurs est configurée pour ajuster une ou plusieurs caractéristiques d'environnement de l'environnement autour du lit intelligent multifonctionnel basé sur la commande provenant du contrôleur. Par exemple, la ou les caractéristiques d'environnement comprennent au moins une des caractéristiques suivantes : la lumière autour du lit intelligent multifonctionnel, le son autour du lit intelligent multifonctionnel, la température autour du lit intelligent multifonctionnel, l'humidité autour du lit intelligent multifonctionnel ou la qualité de l'air autour du lit intelligent multifonctionnel.

Dans certains exemples, le contrôleur est configuré pour déterminer la présence d'un ou plusieurs utilisateurs dans le lit intelligent multifonctionnel sur la base d'une entrée provenant des capteurs de force dans chacun de la pluralité des pieds de lit. Plus spécifiquement, le contrôleur est en outre configuré pour déterminer l'identité du ou des utilisateurs dans le lit intelligent multifonctionnel. Par exemple, le contrôleur comprend un classificateur de sons, configuré pour déterminer l'identité du ou des utilisateurs dans le lit intelligent multifonctionnel. Dans certains exemples, l'identité du ou des utilisateurs dans le lit intelligent multifonctionnel est déterminée sur la base d'une entrée provenant des capteurs de force dans chacun de la pluralité des pieds du lit.

Dans certains exemples, le lit intelligent multifonctionnel comprend en outre un capteur de balistocardiographie, couplée en communication au contrôleur et configurée pour déterminer au moins l'un des paramètres suivants : une fréquence cardiaque, une fréquence respiratoire ou une fréquence de retournement au lit d'un ou plusieurs utilisateurs dans le lit multifonctionnel intelligent, dans lequel le contrôleur est configuré pour faire correspondre à au moins l'un des paramètres suivants, la fréquence cardiaque, la fréquence respiratoire ou la fréquence de retournement, l'identité de l'utilisateur ou des utilisateurs dans le lit intelligent multifonctionnel.

Dans certains exemples, la pluralité de capteurs environnementaux comprend de multiples microphones pour différencier le ronflement de multiples utilisateurs du lit intelligent multifonctionnel. Selon l'invention, la pluralité de capteurs environnementaux comprend un ou plusieurs microphones, configurés pour déformer un son réel, obtenu autour du lit intelligent multifonctionnel générant ainsi un son déformé, non reconnaissable pour un être humain.

Dans certains exemples, le contrôleur reçoit le son déformé du ou des microphones et n'a pas accès au son réel. Par exemple, le contrôleur est configuré pour identifier une ou plusieurs des catégories sonores suivantes dans le son déformé: une conversation normale, une conversation rieuse et joyeuse, des disputes et des cris, et des actes intimes. Par exemple, le contrôleur est configuré pour analyser collectivement la ou les catégories de sons identifiées ensemble avec les entrées reçues des capteurs de force dans chacun de la pluralité des pieds du lit. Dans certains exemples, le contrôleur est configuré pour donner une note à la relation intime basée sur au moins le son déformé.

L'invention concerne également un procédé de fonctionnement d'un lit intelligent multifonctionnel. Dans certains exemples, le procédé comprend la réception de données provenant des capteurs de force dans les pieds du lit intelligent multifonctionnel, le traitement des données reçues des capteurs de force, l'utilisation d'un contrôleur du lit intelligent multifonctionnel et l'ajustement d'au moins une ou plusieurs caractéristiques du lit intelligent multifonctionnel en utilisant une pluralité d'actionneurs commandés par les entrées provenant du contrôleur.

Selon l'invention, le procédé comprend en outre l'obtention d'un son réel autour du lit intelligent multifonctionnel en utilisant un ou plusieurs microphones du lit intelligent multifonctionnel et, en utilisant le ou les microphones, la distorsion du son réel générant ainsi un son déformé. Le procédé comprend en outre la fourniture du son déformé au contrôleur du lit intelligent multifonctionnel. Par exemple, le ou les microphones isolent le contrôleur du lit intelligent multifonctionnel du son réel autour du lit intelligent multifonctionnel. Dans certains exemples, les données, reçues des capteurs de force, sont traitées par le contrôleur avec le son déformé pour générer la commande pour ajuster au moins une ou plusieurs caractéristiques du lit intelligent multifonctionnel.

Dans certains exemples, le procédé comprend en outre l'obtention de données du ballistocardiogramme à partir d'un capteur de balistocardiographie du lit intelligent multifonctionnel, dans lequel les données, reçues des capteurs de force, sont traitées par le contrôleur conjointement avec les données du ballistocardiogramme pour générer la commande pour l'ajustement d'au moins une ou plusieurs caractéristiques du lit intelligent multifonctionnel.

### BREVE DESCRIPTION DES SCHEMAS

La Fig. 1A est une vue latérale schématique d'un lit intelligent multifonctionnel, conformément à quelques exemples.
La Fig. 1B est une vue de dessus schématique du lit intelligent multifonctionnel de la Fig. 1A avec le matelas retiré, conformément à quelques exemples.
La Fig. 1C est une vue schématique en perspective d'un cadre du lit intelligent multifonctionnel de la Fig. 1A, conformément à quelques exemples.
La Fig. 2 est un schéma synoptique schématique de divers composants du lit intelligent multifonctionnel, conformément à quelques exemples.
La Fig. 3 est une vue latérale schématique d'un lit intelligent multifonctionnel, illustrant un capteur de balistocardiographie (BCG), intégrée dans le matelas du lit intelligent multifonctionnel, conformément à quelques exemples.
La Fig. 4 est une vue latérale schématique d'un lit intelligent multifonctionnel, illustrant de multiples unités d'ajustement de la fermeté du matelas réparties dans tout le matelas du lit intelligent multifonctionnel, conformément à quelques exemples.
La Fig. 5 est un organigramme de processus correspondant à un procédé de fonctionnement d'un lit intelligent multifonctionnel, conformément à certains modes de réalisation.
La Fig. 6 est un organigramme de processus correspondant à une méthode de classification d'un son modifié, conformément à quelques exemples.
La Fig. 7 est un schéma synoptique du contrôleur du lit intelligent multifonctionnel, conformément à quelques exemples.

### DESCRIPTION DETAILLEE

Dans la description qui suit, de nombreux détails spécifiques sont exposés afin de fournir une compréhension approfondie des concepts présentés. Dans certains exemples, les concepts présentés sont mis en pratique sans certains ou tous ces détails spécifiques. Dans d'autres cas, des opérations de processus bien connues n'ont pas été décrites en détail afin de ne pas obscurcir inutilement les concepts décrits. Bien que certains concepts soient décrits en conjonction avec les exemples spécifiques, on comprendra que ces exemples ne sont pas destinés à être limitatifs.

### Introduction

Les lits intelligents multifonctionnels et leurs procédés de fonctionnement fournissent diverses fonctions nouvelles, qui ne sont pas disponibles dans les lits conventionnels. Certains exemples de ces fonctions comprennent, mais sans s'y limiter, la surveillance de la santé, la gestion du sommeil, les ajustements de dureté du matelas et les fonctions de suivi des rapports intimes. Ces fonctions sont fournies de manière automatisée à l'aide de divers capteurs et actionneurs des lits intelligents. Dans certains exemples, la contribution des utilisateurs est minime ou complètement absente.

Dans certains exemples, un lit intelligent est équipé de capteurs de force, qui sont couplées ou intégrées dans les pieds du lit intelligent. Chaque capteur de force mesure en continu le poids appliqué au pied correspondant, ce qui permet de surveiller les changements de poids des utilisateurs (par exemple, les gains / pertes de poids), la répartition du poids (par exemple, les mouvements au lit, la position dans le lit), les heures d'entrée et de sortie du lit (par exemple, l'éveil nocturne), etc. Les données de poids sont fournies à un contrôleur, qui agrège et analyse ces données pour relever diverses caractéristiques. Par exemple, une analyse de données peut être utilisée pour générer des rapports pour les utilisateurs (par exemple, profil de poids, profil de sommeil) et / ou contrôler divers actionneurs du lit intelligent (par exemple, changer l'orientation de différentes parties du cadre, changer la dureté des différentes parties di matelas, allumer / éteindre la lumière).

Dans certains exemples, le contrôleur du lit intelligent comprend divers modules, spécifiquement configurés pour fournir diverses fonctions du lit intelligent. Par exemple, le contrôleur peut comprendre un module environnemental, configuré pour recevoir une entrée de capteurs environnementaux (par exemple, des microphones, des capteurs de lumière, des thermomètres) et pour contrôler des ajusteurs d'environnement (par exemple, des haut-parleurs, des lumières, des appareils de chauffage). Le module environnemental est également configuré pour interagir avec un ou plusieurs autres modules du contrôleur.

Un autre exemple de module est un module de dureté de matelas, qui est configuré pour contrôler et ajuster la dureté du matelas ou, plus spécifiquement, la dureté de différentes parties de matelas. Encore un autre exemple des modules de contrôleur est un module de surveillance de la santé, qui est configuré pour recevoir une entrée de divers capteurs, y compris des capteurs de santé spécifiques, tels qu'un capteur de balistocardiographie (BCG). Le module de surveillance de la santé est configuré pour fournir des rapports aux utilisateurs sur leurs conditions de santé attendues (par exemple, profil de poids, efficacité du sommeil, fréquence cardiaque (FC), fréquence respiratoire (FR), fréquence de retournement au lit (RT), etc.).

Dans certains exemples, le contrôleur du lit intelligent comprend un module de gestion du sommeil, qui reçoit des données de capteur et une sortie d'autres modules liés à la qualité du sommeil. Le module de gestion du sommeil est également configuré pour contrôler divers actionneurs, par exemple pour améliorer la qualité du sommeil. Par exemple, le module de gestion du sommeil peut régler la lumière, la température et / ou le son de l'environnement du lit. Dans certains exemples, le module de gestion du sommeil demande au module de dureté du matelas d'ajuster la dureté d'une ou plusieurs sections du matelas.

Dans certains exemples, le contrôleur de lit intelligent comprend un module d'intimité, qui est configuré pour recevoir et analyser des données préfiltrées d'un ou plusieurs capteurs, tels que des microphones ou des capteurs de force. Le pré-filtrage des données des capteurs avant que ces données ne soient analysées et stockées permet de répondre à divers problèmes de confidentialité. Par exemple, un modificateur de son est utilisé au niveau du microphone pour supprimer certains types de sons et / ou pour rendre divers et, dans certains exemples, même tous les sons méconnaissables pour les humains (pour la vie privée). Cependant, le son modifié conserve encore suffisamment d'informations distinctives et / ou discriminantes permettant à l'algorithme de classification sonore d'effectuer diverses opérations, telles que la séparation de plusieurs sources vocales, la reconnaissance vocale, l'analyse des sentiments, etc. En d'autres termes, au lieu de supprimer des parties du son, le son est modifié / déformé d'une ou plusieurs manières pour rendre le son modifié méconnaissable aux humains. Selon l'invention, le son modifié contient toujours des caractéristiques distinctives permettant au classificateur d'intelligence artificielle (IA) de différencier les différentes classes de son. Le contenu global du son est perdu lors de la modification du son tandis que diverses caractéristiques de différenciation essentielles sont conservées. Il est à noter que la modification sonore est irréversible pour préserver la confidentialité de données personnelles intimes. En tant que tel, le son modifié ne peut pas être reconverti en son d'origine. La fonctionnalité irréversible est obtenue en traitant le son original à l'aide d'une fonction mathématique irréversible. En conséquence, le son modifié peut être stocké et partagé sans souci de confidentialité.

### Exemples du Lit Intelligent

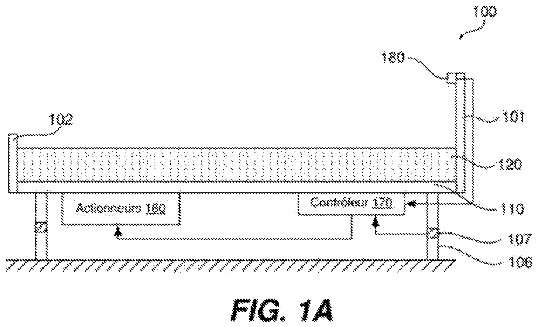

La Fig. 1A est une vue latérale schématique du lit intelligent 100, conformément à quelques exemples. Le lit intelligent 100 est également appelé lit intelligent multifonction, en raison de diverses fonctions nouvelles décrites ci-dessous. Le lit intelligent 100 comprend le cadre 110 et le matelas 120, supportés par le cadre 110. Le lit intelligent 100 comprend également une pluralité de pieds 106, de sorte que chaque pluralité de pieds 106 comprend ou est couplée à un capteur de force 107. La pluralité de pieds 106 maintient le cadre 110 et le matelas 120 au-dessus du niveau du sol.

Les capteurs de force 107 sont configurées pour mesurer le poids appliqué à chacun des pieds du lit 106. La sortie des capteurs de force 107 peut être utilisée pour déterminer divers changements de poids associés à l'utilisation du lit intelligent 100 (par exemple, un utilisateur entrant ou sortant du lit intelligent 100), répartition du poids du lit intelligent 100. Dans certains exemples, ces mesures et analyses de poids sont effectuées en continu, ce qui permet de déterminer les changements dynamiques du poids des utilisateurs, les mouvements au lit (par exemple, mesurer les changements dans la distribution du poids). Dans l'ensemble, les capteurs de force 107 permettent de déterminer quand une personne s'allonge dans le lit, quitte le lit, se déplace pendant qu'elle est dans le lit, etc. La sortie des capteurs de force 107 est fournie et utilisée par le contrôleur 170.

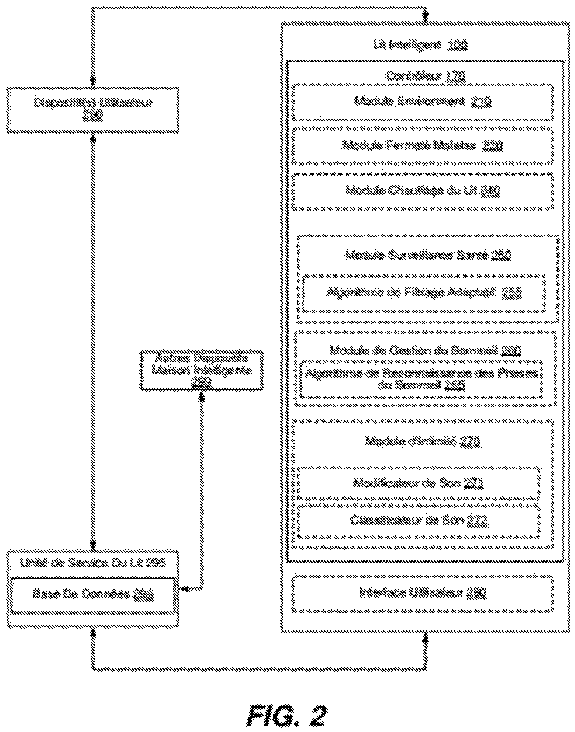

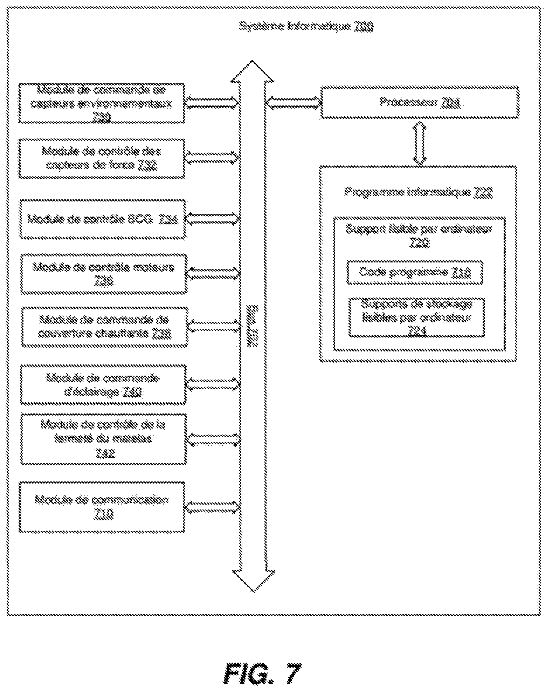

Le lit intelligent 100 comprend également un contrôleur 170, qui est configuré pour recevoir une entrée de divers capteurs (par exemple, des capteurs d'environnement 180, des capteurs de force 107) et pour fournir des instructions aux actionneurs 160. Plus précisément, le contrôleur 170 est couplé en communication aux capteurs de force 107. dans chacune d'une pluralité de pieds 106 et à chaque capteur d'environnement 180. Les actionneurs 160 sont également couplés en communication au contrôleur 170 et configurés pour changer une ou plusieurs caractéristiques du lit intelligent multifonctionnel 100, basé sur l'entrée du contrôleur 170. Des caractéristiques supplémentaires du contrôleur 170 sont décrites ci-dessous en référence à la Fig. 2 et la fig. 7.

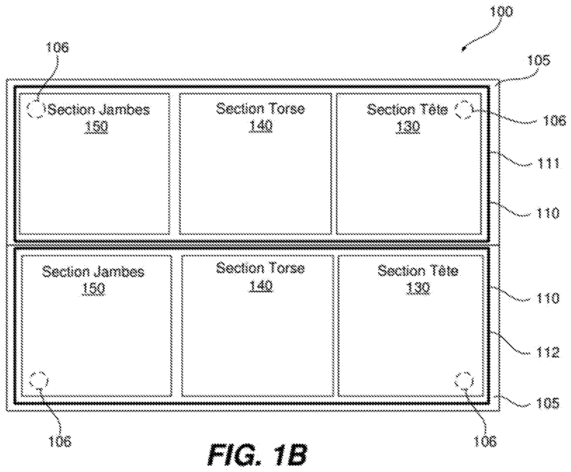

La Fig. 1B est une vue de dessus schématique du lit intelligent 100, conformément à quelques exemples. Dans ces exemples, le lit intelligent 100 comprend deux cadres 110, par exemple le premier cadre 111 et le deuxième cadre 112. Dans d'autres exemples, le lit intelligent 100 comprend un seul cadre 110 ou comprend un ou plusieurs cadres supplémentaires 110 (par exemple un troisième cadre). La pluralité de pieds 106 fournit un support à la fois au premier cadre 111 et au deuxième cadre 112. Par exemple, le lit intelligent 100 comprend en outre une ou plusieurs bases 105, fournissant un support à chacun du premier cadre 111 et du deuxième cadre 112 et agissant comme une structure intermédiaire entre chacun du premier cadre 111 et second cadre 112 et pluralité de pieds 106. Par exemple, chacun du premier cadre 111 et du second cadre 112 a une base dédiée 105 et est couplé de manière réglable à cette base 105.

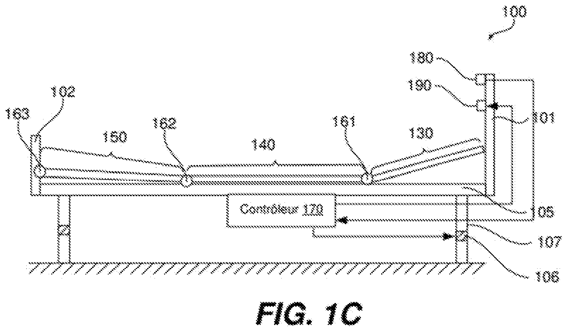

Dans certains exemples, chaque cadre est couplé de manière réglable à la base 105. Spécifiquement, chacun du premier cadre 111 et du deuxième cadre 112 comprend plusieurs sections, qui sont indépendamment mobiles, par exemple par rapport à la base 105 et / ou à la pluralité de pieds 106. En se référant aux Fig. 1B et 1C, chacun du premier cadre 111 et du second cadre 112 comprend une section de tête 130, une section de torse 140 et une section de jambes 150. Divers exemples avec moins ou plus de sections sont également compris dans l'étendue de champ d'application de l'invention. La section de tête 130, la section de torse 140 et la section de jambes 150 dans chaque cadre sont réglables indépendamment l'une de l'autre (dans le même cadre et à partir des sections correspondantes dans un autre cadre). Par exemple, la section de tête 130 du premier cadre 111 est réglable indépendamment de la section de tête 130 du deuxième cadre 112. Ces réglages peuvent impliquer un angle de la section correspondante par rapport à la base 105 et / ou par rapport à une ou plusieurs autres sections, qui peuvent être appelés angles d'inclinaison. Les différents ajustements de chaque section et les raisons de ces ajustements sont décrits ci-dessous.

Selon l'invention, le lit intelligent 100 comprend une pluralité d'actionneurs 160. Un exemple d'actionneurs est des moteurs utilisés, par exemple, pour changer l'orientation d'au moins l'une des parties suivantes : la section de tête 130, la section de torse 140 et / ou la section des jambes 150 par rapport à la base 105. La Fig. 1C illustre un exemple de lit intelligent 100 comprenant un premier entraînement 161 pour changer l'orientation de la section de tête 130, un deuxième entraînement 162 pour changer l'orientation de la section de torse 140 et un troisième entraînement 163 pour changer l'orientation de la section de jambes 150. Chaque entraînement est indépendamment commandé par le contrôleur 170. Outre les actionneurs mécaniques, tels que les entraînements, les actionneurs 160 peuvent comprendre d'autres types tels que des actionneurs pour ajuster l'environnement, qui peuvent être appelés ajusteurs d'environnement 190, qui sont décrits ci-dessous.

En se référant à la Fig. 1A, selon l'invention, le lit intelligent 100 comprend également une pluralité de capteurs environnementaux 180. Divers composants du lit intelligent 100 peuvent être utilisés pour supporter des capteurs environnementaux 180, tels que le cadre 110, la tête de lit 101, le pied de lit 102, et similaires. Dans des exemples plus spécifiques, au moins un ou plusieurs capteurs environnementaux 180 sont positionnés sur la tête de lit 101, par exemple, à la position la plus élevée possible de la tête de lit 101. En général, la position des capteurs environnementaux 180 dépend du type de détection. Par exemple, des microphones peuvent être placés sur des côtés opposés de la tête de lit 101 (par exemple, un à droite et un à gauche) pour assurer la différenciation sonore de différentes personnes dans le lit intelligent 100. En outre, même le même capteur peut être placé à plusieurs différents emplacements, par exemple un microphone dans la tête de lit 101, tandis que les circuits de traitement du son se trouvent à un autre emplacement.

Les capteurs environnementaux 180 sont configurés pour mesurer la température, l'humidité, la qualité de l'air, la pression atmosphérique, le niveau de lumière, le niveau de bruit et / ou d'autres caractéristiques dans l'environnement entourant le lit intelligent 100. Les capteurs environnementaux 180 peuvent être positionnés à divers emplacements relatifs. au cadre 110 et / ou au matelas 120. Dans le même exemple ou dans d'autres exemples, les capteurs d'environnement 180 comprennent un ou plusieurs thermomètres, par exemple positionnés à divers niveaux de hauteur ou parties du lit pour déterminer la distribution de température dans l'environnement du lit. Il convient de noter que le lit intelligent 100 peut comprendre divers autres capteurs, se concentrant sur des paramètres utilisateur, qui sont décrits plus en détail ci-dessous. Un autre exemple de capteurs environnementaux 180 est un capteur de lumière, configuré pour détecter l'intensité de la lumière dans l'environnement du lit. La sortie des capteurs environnementaux 180 est fournie au contrôleur 170.

Dans certains exemples, les capteurs d'environnement 180 sont positionnés à proximité ou fixés à la section de tête 130, par exemple positionnés dans la tête de lit 101. Par exemple, les capteurs d'environnement 180 comprennent un microphone pour mesurer à la fois le bruit ambiant et le bruit produit par une personne en smart lit 100 (par exemple, ronflement). Dans des exemples plus spécifiques, les capteurs d'environnement 180 comprennent deux microphones, chacun positionné dans la tête de lit 101. En tant que tel, un microphone est utilisé par une personne (par exemple, positionné sur le premier cadre 111), tandis que l'autre microphone est utilisé pour capturer les sons d'une autre personne. (par exemple, positionné sur le deuxième cadre 112). Ce type d'arrangement est utilisé, par exemple, pour détecter laquelle de deux personnes ronfle réellement.

Dans certains exemples, le lit intelligent 100 comprend un ou plusieurs ajusteurs d'environnement 190, qui sont des exemples spécifiques d'actionneurs 160. Les ajusteurs d'environnement 190 peuvent être configurés pour ajuster la température, l'humidité, le niveau de lumière, le niveau de bruit et / ou d'autres caractéristiques dans l'environnement entourant le lit intelligent 100. Certains exemples d'ajusteurs d'environnement 190 comprennent, mais sans s'y limiter, les appareils de chauffage, les ventilateurs, les humidificateurs, les lumières, les haut-parleurs, et analogues. Les ajusteurs d'environnement 190 sont commandés sur la base de l'entrée du contrôleur 170. Par exemple, le contrôleur 170 peut ordonner aux ajusteurs d'environnement 190 de changer un ou plusieurs paramètres environnementaux sur la base de l'entrée des capteurs environnementaux 180 et / ou des déterminations effectuées par un ou plusieurs modules du contrôleur 170, qui sont décrites ci-dessous.

En se référant à la Fig. 2, le lit intelligent 100 est couplé de manière communicative à un ou plusieurs dispositifs d'utilisateur 290 et / ou à l'unité de service du lit intelligent 295. Certains exemples de dispositifs d'utilisateur 290 comprennent, mais sans s'y limiter, un smartphone, une montre intelligente, une tablette, un ordinateur portable, un ordinateur de bureau, centres / hubs pour maison intelligente, etc. Les dispositifs utilisateurs 290 peuvent être couplés en communication au lit intelligent 100 soit directement, par exemple via le protocole Bluetooth, soit indirectement, par exemple via des réseaux locaux et / ou distants (Wi-Fi, Internet). Le contrôleur 170 est configuré pour fournir aux dispositifs utilisateurs 290 divers capteurs et autres données similaires, obtenus par le contrôleur 170. Dans certains exemples, le contrôleur 170 est configuré pour générer des rapports basés sur ces données, et les rapports sont fournis aux dispositifs utilisateurs 290. En outre, le contrôleur 170 est configuré pour recevoir diverses entrées des dispositifs d'utilisateur 290, par exemple, pour modifier divers paramètres du lit intelligent 100.

Dans certains exemples, l'unité de service du lit intelligent 295 est un serveur distant d'arrière-plan, couplé en communication au lit intelligent 100 via au moins des réseaux distants (par exemple, Internet). Par exemple, l'unité de service du lit intelligent 295 est un serveur hébergé par un fabricant de lit intelligent 100 pour obtenir des données sur diverses conditions de fonctionnement du lit intelligent 100, par exemple pour lancer une requête de service / réparation, mettre à jour la fonctionnalité (par exemple, mises à jour en direct), etc. Dans certains exemples, la base de données 296 stocke divers paramètres pour le lit intelligent 100, qui sont automatiquement téléchargés et utilisés par le lit intelligent 100 et, dans certains exemples, par d'autres appareils autour du lit intelligent 100, par exemple les appareils domestiques intelligents 299. En outre, dans certains exemples, l'unité de service du lit intelligent 295 crée des paramètres pour le lit intelligent 100, sur la base d'une entrée provenant de plusieurs autres lits intelligents, effectuant ainsi une exploration et une analyse de données. Enfin, l'unité de service du lit intelligent 295 peut télécharger des paramètres pour un utilisateur spécifique lorsque cet utilisateur voyage et dort dans d'autres lits intelligents, par exemple, un hôtel, un hôpital, une résidence secondaire, etc.

### Exemples du Contrôleur de Lit

Comme le montre la Fig. 2, le contrôleur 170 du lit intelligent 100 comprend divers modules, configurés pour fournir diverses fonctions du lit intelligent 100. Certains exemples de ces modules sont le module d'environnement 210, le module de fermeté du matelas 220, le module de chauffage de lit 240, le module de surveillance de la santé 250, le module de gestion du sommeil 260 et le module d'intimité 270. Une personne du métier comprendra que le contrôleur 170 peut avoir moins de modules et / ou de modules supplémentaires, permettant diverses fonctions décrites dans cette divulgation. Ces modules peuvent être activés dans le matériel et / ou le logiciel comme décrit plus en détail ci-dessous en référence à la Fig. 7.

En se référant à la Fig. 2, dans certains exemples, le contrôleur 170 comprend également une interface utilisateur 280, qui peut être utilisée par un utilisateur (par exemple, pendant que l'utilisateur est dans le lit intelligent 100) pour contrôler diverses fonctions du lit intelligent 100 et / ou afficher diverses données, recueillies par le contrôleur 170. Certaines fonctions de l'interface utilisateur 280 sont les mêmes que celles des dispositifs utilisateur 290, décrits ci-dessus. En d'autres termes, une personne peut utiliser le dispositif utilisateur 290 ou l'interface utilisateur 280 pour contrôler le lit intelligent 100 et examiner diverses données. Dans certains exemples, l'interface utilisateur 280 comprend un ensemble dédié de dispositifs d'entrée (par exemple, des commutateurs, des cadrans) pour commander des fonctions spécifiques du lit intelligent 100. Dans le même exemple ou dans d'autres exemples, l'interface utilisateur 280 comprend un écran tactile reconfigurable. Par exemple, un utilisateur peut régler manuellement diverses caractéristiques du lit intelligent 100 via l'interface utilisateur 280. Ces caractéristiques réglées manuellement peuvent être combinées avec des caractéristiques déterminées automatiquement (par divers modules du contrôleur 170, décrits ci-dessus). Par exemple, des caractéristiques réglées manuellement peuvent être utilisées pour remplacer les caractéristiques déterminées automatiquement ou pour ajuster davantage les caractéristiques déterminées automatiquement. Certains exemples de caractéristiques réglées manuellement comprennent, mais sans s'y limiter, la couleur des lumières, son intensité, le niveau sonore de la musique, la vitesse des moteurs de réglage de position, et autres.

### Exemples du Module Environnent

Dans certains exemples, le contrôleur 170 comprend le module d'environnement 210. Le module d'environnement 210 est configuré pour recevoir des données relatives à l'environnement entourant le lit intelligent 100, par exemple à partir des capteurs environnementaux 180. Ce type de données peut être appelé données d'environnement du lit et peut inclure la température, l'humidité, le bruit, etc. Le module d'environnement 210 est configuré pour partager sélectivement ces données d'environnement du lit avec d'autres modules, comme avec le module de gestion de sommeil 260. Par exemple, le module de gestion de sommeil 260 est configuré pour corréler les données d'environnement du lit avec la qualité de sommeil de l'utilisateur, qui peut être présentée comme un score de sommeil. La fonctionnalité du module de gestion du sommeil 260 est décrite ci-dessous.

Dans certains exemples, le module d'environnement 210 commande les ajusteurs d'environnement 190 et / ou d'autres dispositifs de maison intelligente 299 pour obtenir des caractéristiques d'environnement souhaitées, par exemple, déterminées par le module d'environnement 210 ou d'autres modules. Par exemple, le module d'environnement 210 peut déterminer les caractéristiques d'environnement souhaitées sur la base du réglage actuel (fourni par l'utilisateur), des tendances historiques (par exemple, des paramètres appris), de l'identité de l'utilisateur (par exemple, déterminée à partir du poids, du profil sonore), et / ou d'autres facteurs similaires.

Dans certains exemples, le module d'environnement 210 est configuré pour contrôler le niveau de bruit autour du lit intelligent 100 ou, plus spécifiquement, autour de la partie tête de chaque utilisateur. Par exemple, les ajusteurs d'environnement 190 peuvent comprendre un ou plusieurs haut-parleurs ou écouteurs commandés par le module d'environnement 210 pour générer un son spécifique (par exemple, des sons de réglage de l'humeur, relaxation, méditation), un bruit blanc, une annulation de bruit, etc.

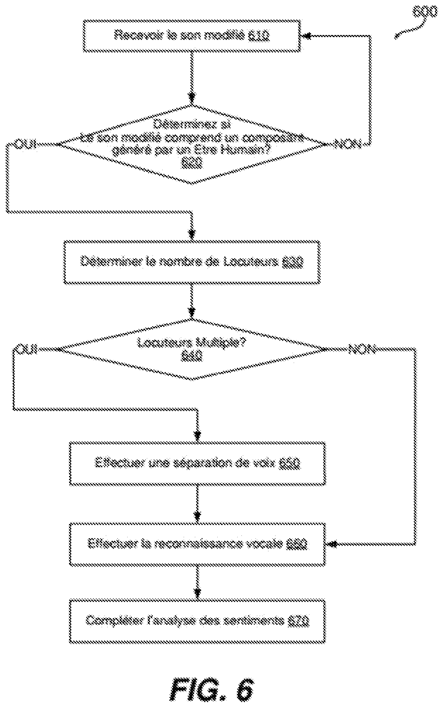

Dans certains exemples, les données sonores reçues par le module d'environnement 210 représentent une version modifiée / déformée du son réel dans l'environnement, pour assurer la confidentialité des sons émis par l'utilisateur. La distorsion peut être effectuée par un matériel analogique, par exemple, un circuit analogique dans le module d'environnement 210. Le traitement du son modifié / déformé (par exemple, la classification du son) est décrit ci-dessous en référence à la Fig. 6.

Dans certains exemples, le contrôleur 170 utilise une entrée sonore pour contrôler les unités de fermeté 400. Par exemple, un lit intelligent 100 avec deux cadres peut avoir deux microphones. Les deux microphones sont utilisés pour détecter la personne qui ronfle, en comparant l'amplitude du son dans les deux microphones. Cette comparaison permet de déterminer de quel côté se trouve la personne qui ronfle. Dans certains exemples, le son de ronflement est détecté par un modèle d'intelligence artificielle (AI) de réseau neuronal convolutif d'apprentissage supervisé (CNN), formé pour différencier le ronflement des sons non ronflants. Dans certains exemples, le signal sonore est modifié au niveau du matériel de réception, par exemple un microphone, pour protéger la vie privée de l'utilisateur.

Dans certains exemples, le module d'environnement 210 est configuré pour contrôler le niveau de lumière autour du lit intelligent 100 ou, plus spécifiquement, autour de chaque utilisateur. Par exemple, le module d'environnement 210 peut recevoir un signal des capteurs de force 107 indiquant qu'un utilisateur se lève du lit. Dans ce cas, le module d'environnement 210 ordonne à une lumière de s'allumer pour aider l'utilisateur à naviguer dans l'environnement autour du lit intelligent 100.

Dans certains exemples, si une ou plusieurs conditions d'environnement ne peuvent pas être contrôlées par les ajusteurs d'environnement 190, alors le module d'environnement 210 fournit un rapport (à l'utilisateur), recommandant divers ajustements à l'environnement, par exemple, l'installation de solutions d'isolation acoustique dans la chambre à coucher, le déplacement du lit intelligent 100 vers un nouvel emplacement, la fermeture des volets. Comme décrit ci-dessous, dans certains exemples, le lit intelligent 100 est couplé de manière communicative à d'autres dispositifs domestiques intelligents 299, tels que des thermostats intelligents, des lumières intelligentes, des volets intelligents et similaires. Dans ces exemples, la sortie des ajusteurs d'environnement 190 est utilisée pour commander un ou plusieurs de ces dispositifs, sans intervention directe de l'utilisateur.

Globalement, le module d'environnement 210 est configuré pour recevoir et traiter divers types de données d'environnement (température, humidité, bruit, etc.) et, dans certains exemples, pour corréler ces données avec d'autres types de données (par exemple, qualité du sommeil, conditions de santé, activités, etc.). Le module d'environnement 210 est configuré pour déterminer les paramètres d'environnement cible et pour contrôler les ajusteurs d'environnement 190 et / ou les composants externes responsables du maintien de l'environnement autour du lit intelligent 100. Dans certains exemples, le module d'environnement 210 est configuré pour générer un rapport, lié aux conditions d'environnement et recommandations pour modifier ces conditions d'environnement et pour envoyer ce rapport au dispositif d'utilisateur 290.

### Exemples du Module Chauffage du Lit

Dans certains exemples, le contrôleur 170 comprend le module de chauffage de lit 240. Le module de chauffage de lit 240 est utilisé pour contrôler la puissance appliquée à chacune d'une ou plusieurs couvertures chauffantes, oreillers chauffants, draps chauffants et / ou matelas chauffants du lit intelligent 100, par exemple, sur la base de diverses entrées reçues par le contrôleur 170 et / ou sur la base de diverses déterminations par le contrôleur 170. Le chauffage du matelas doit être distingué du réglage de la fermeté du matelas, en utilisant un élément chauffant intégré dans le matelas, comme décrit plus en détail ci-dessous.

Dans certains exemples, les opérations du module de chauffage de lit 240 sont synchronisées avec les opérations du module d'environnement 210, qui peut contrôler la température des environnements. Par exemple, le module de chauffage de lit 240 peut activer la couverture chauffante lorsque le module d'environnement 210 permet à la température ambiante de baisser.

### Exemples d'Ajustage de la Fermeté du Matelas

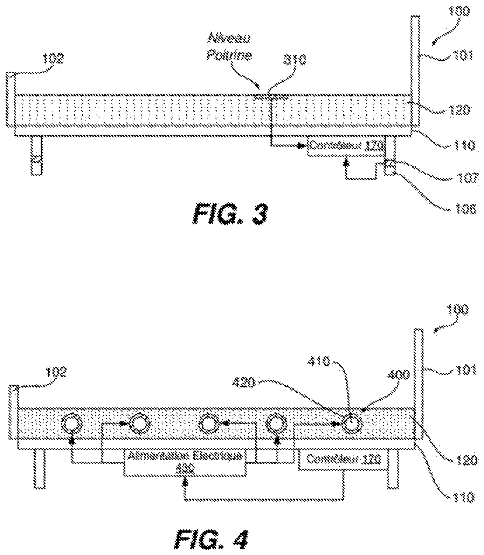

Dans certains exemples, le contrôleur 170 comprend un module de fermeté du matelas 220. Le module de fermeté du matelas 220 est utilisé pour contrôler et ajuster la dureté du matelas 120, par exemple sur la base d'une entrée provenant de capteurs ou d'autres modules, par exemple, le module de gestion du sommeil 260, le module d'environnement 210 et analogues. Par exemple, le module de gestion du sommeil 260 peut être configuré pour ajuster automatiquement la dureté du matelas 120 pour améliorer la qualité globale du sommeil, par exemple, représentée par le score de sommeil comme décrit plus en détail ci-dessous. Dans certains exemples, le module de fermeté du matelas 220 est utilisé pour contrôler la dureté de différentes parties du matelas 120, comme cela sera maintenant décrit en référence à la Fig. 4.

La Fig. 4A est une vue en coupe schématique du lit intelligent 100, montrant plusieurs unités de fermeté 400, intégrées dans le matelas 120. Chaque unité de fermeté 400 comprend une base en mousse viscoélastique 410 et une unité de chauffage 420. Dans certains exemples, l'unité de chauffage 420 est configurée comme une chemise / coque entourant la base en mousse viscoélastique 410. L'unité de chauffage 420 peut être un élément chauffant à résistance électrique, avec un niveau de température contrôlé par le module de dureté du matelas 220 ou, plus généralement, par le contrôleur 170.

La base de mousse viscoélastique 410 est formée à partir d'une mousse de polyuréthane viscoélastique avec une formulation adaptée. En dessous d'une certaine température de consigne de la mousse (liée à la température de transition vitreuse de la mousse de polyuréthane viscoélastique), la base de mousse viscoélastique 410 a un niveau de dureté défini. Lorsque la température de la base de mousse viscoélastique 410 augmente en raison du chauffage par l'unité de chauffage 420 et dépasse la température de consigne de la mousse, la dureté de la base de mousse viscoélastique 410 diminue.

Pour les besoins de cette divulgation, la transition vitreuse est définie comme une plage de températures, au cours de laquelle le polymère passe d'un état dur et vitreux à un matériau souple et caoutchouteux. Contrairement à la température de fusion (qui est une propriété thermodynamique du matériau solide), la température de transition vitreuse est une propriété dynamique des polymères amorphes et est liée au comportement de relaxation des segments de chaîne polymère locaux. En conséquence, la température de transition vitreuse dépend des facteurs suivants affectant la mobilité des segments de chaîne polymère: (1) densité de réticulation dans la structure du réseau, (2) aromaticité (par exemple, définie comme une fraction pondérale des structures aromatiques dans le matrice polymère), (3) concentration de plastifiant et autres facteurs similaires.

Dans certains exemples, la température de transition vitreuse de la mousse de polyuréthane viscoélastique formant la base de mousse viscoélastique 410 est comprise entre + 25 ° C et + 40 ° C. Cette température est beaucoup plus élevée que celle utilisée classiquement. Une telle température de transition vitreuse élevée est obtenue en (1) augmentant la densité de réticulation en augmentant le rapport isocyanates sur polyol (appelé «indice»), (2) faisant varier la composition chimique des segments de réseau en faisant varier les longueurs de chaîne des polyols et les monomères, et (3) l'augmentation de la teneur en urée et en poly-urée de la mousse en augmentant la teneur en eau de la formulation.

Dans certains exemples, la mousse de polyuréthanne viscoélastique est formée en utilisant un récipient étroitement fermé dans lequel la mousse est mise à réagir. Le gaz de dioxyde de carbone (CO2) résultant généré par la réaction est empêché de s'échapper du récipient fermé, mais de manière contrôlée. Plus précisément, le gaz généré à l'intérieur du récipient fermé exerce une force mécanique répartie de manière homogène qui agit contre l'expansion de la mousse et empêche donc la diminution de la densité. Moins il y a de gaz s'échappant de la chambre de moussage, plus la densité est élevée. Plus le gaz autorisé à s'échapper par une vanne de régulation est élevé, plus la densité est faible. Ce nouveau procédé de moussage permet la production d'une mousse de polyuréthane viscoélastique à haute densité, à haute teneur en poly-urée tout en conservant une densité élevée qui dans certains exemples varie entre 35 kg / m3 et 75 kg / m3 ou plus. Dans certains exemples, la densité est comprise entre 55 kg / m3 et 65 kg / m3 pour supporter des charges élevées et des déformations importantes notamment pendant le mode chauffé pendant lequel la mousse présente ses propriétés molles et est donc plus sujette à la déformation. En outre, la mousse a une teneur élevée en poly-urée en raison du niveau élevé de teneur en eau dans la formulation.

Dans certains exemples, la mousse est expansée dans une multitude de formes de moules pour donner la forme désirée. En outre, dans certains exemples, la mousse est usinée (par exemple, en utilisant un outil d'usinage à commande numérique par ordinateur (CNC)) à la forme souhaitée à partir d'un bloc de départ. Le pain en mousse découpé est ensuite inséré dans une chemise chauffée électriquement ayant plusieurs éléments chauffants dans le côté intérieur de la chemise adjacente au pain en mousse et un matériau isolant thermiquement (par exemple, une feuille d'aluminium) sur le côté extérieur afin d'éviter la perte d'énergie thermique et aussi pour éviter que les composants qui entreraient en contact avec l'appareil ne soient chauffés.

Dans certains exemples, plusieurs unités de fermeté 400 sont positionnées dans le même matelas 120 et, plus spécifiquement, réparties le long de la longueur du matelas 120. Par exemple, une paire d'unités de fermeté adjacentes 400 peut être espacée entre 200 millimètres et 500. millimètres. La taille de la section transversale (par exemple, le diamètre de chaque unité de fermeté 400) peut être comprise entre 50 millimètres et 300 millimètres.

Chacune de ces unités de fermeté multiples 400 est contrôlée indépendamment par le contrôleur 170. Plus spécifiquement, le contrôleur 170 peut fournir une entrée à l'alimentation électrique 430, couplée individuellement à chacune des unités de fermeté 400. Différentes formes d'unité de fermeté 400 ou, plus spécifiquement, de la base de mousse viscoélastique 410 sont dans l'étendue du champ d'application de l'invention, telle que cylindrique, rectangulaire, triangulaire, etc.

Dans certains exemples, le contrôleur 170 utilise une entrée sonore pour contrôler les unités de fermeté 400. Par exemple, le lit intelligent 100 avec deux cadres peut avoir deux microphones, un dans la section de tête 130 de chaque cadre. Les deux microphones sont utilisés pour détecter la personne qui ronfle, en comparant l'amplitude du son dans les deux microphones. Cette comparaison permet de déterminer de quel côté se trouve la personne qui ronfle. Dans certains exemples, le son de ronflement est détecté par un modèle d'intelligence artificielle (IA) de réseau neuronal à convolution d'apprentissage supervisé (CNN), entraîné à différencier le ronflement des sons non ronflants. Dans certains exemples, le signal sonore est modifié au niveau du matériel de réception, par exemple un microphone, pour protéger la vie privée de l'utilisateur.

En outre, dans certains exemples, lors de la détection du ronflement, le contrôleur 170 ordonne à un ou plusieurs entraînements de changer la position d'un ou plusieurs des sections suivantes : la section de tête 130, la section de torse 140 et la section des jambes 150. En se référant à la Fig. 1C, dans des exemples spécifiques, le contrôleur 170 ordonne au premier entraînement 161 d'augmenter l'angle de la section de tête 130, par rapport au sol, augmentant ainsi la position de la tête du dormeur. Le changement de position de la tête est connu pour arrêter temporairement le ronflement car les voies respiratoires obstruées par les muscles du larynx relâchés sont dégagées par le changement de position de la tête. Chaque fois que le ronflement est repris, la position de la tête est à nouveau déplacée, à vitesse lente (par exemple, à moins de 5 ° par minute) pour éviter de réveiller la personne endormie. Par exemple, élever la tête de l'utilisateur d'environ 20 ° à 30 ° par rapport au plan horizontal ouvre les voies respiratoires nasales et peut empêcher le ronflement. Une autre cause de ronflement peut être une répartition inégale du poids.

### Exemples de Surveillance de la Santé

En se référant à la Fig. 2, dans certains exemples, le contrôleur 170 comprend également un module de surveillance de la santé 250. En outre, en se référant à la Fig. 3, le lit intelligent 100 comprend une ou plusieurs capteurs BCG 310, fixées ou intégrées au matelas contrôleur 120. Le contrôleur 170 ou, plus spécifiquement, le module de surveillance de la santé 250 reçoit une entrée des capteurs BCG 310 et, dans certains exemples, des capteurs de force 107, prévus dans les pieds 106 du lit intelligent 100. Spécifiquement, les capteurs de force 107 assurent une surveillance continue et non intrusive du poids de la personne. Par exemple, une personne n'a pas besoin d'utiliser spécifiquement une balance, qu'on peut souvent oubliée d'utiliser. Une telle surveillance continue du poids est particulièrement importante pour les personnes ayant des problèmes cardiaques et d'hypertension non diagnostiqués, car elles ne sont souvent pas en mesure de remarquer une prise de poids en raison de la rétention d'eau corporelle, qui est considérée comme un signal important de dysfonctionnements cardiovasculaires. La surveillance continue du poids peut fournir diverses tendances de comparaison au jour le jour (p. Ex., Suralimentation un jour donné). Par exemple, le lit intelligent 100 est capable d'effectuer des comparaisons quotidiennes à la même heure de la journée, ce qui est l'une des principales recommandations pour garder un bon enregistrement de poids. En outre, le lit intelligent 100 est capable de détecter les stades précoces de l'obésité, pendant lesquels le contrôle du poids est relativement facile, par rapport aux stades ultérieurs. Dans le même temps, la détection de la prise de poids est plus difficile à ce stade précoce en raison de l'augmentation de poids faible et progressive imperceptible. Le lit intelligent 100 surmonte ce défi grâce à une surveillance continue et cohérente.

Cette caractéristique de mesure de poids du lit intelligent 100 est auto-calibrée. Plus précisément, le contrôleur 170 du lit intelligent 100 ou, plus spécifiquement, le module de surveillance de la santé 250 comprend un algorithme qui permet des mesures de poids précises sans être impacté par différentes configurations du lit intelligent 100, par exemple, comme l'ajout / la suppression de divers objets du lit intelligent 100, par ex. , oreillers ou couvertures. Plus précisément, les capteurs de force 107 surveillent en permanence le poids en conjonction avec une surveillance continue de la présence de l'utilisateur dans le lit intelligent 100, par exemple en utilisant le signal BCG, la détection de bruit, le capteur de mouvement, etc. En d'autres termes, le contrôleur 170 n'est pas induit en erreur par des objets non-vivants ajoutés / supprimés du lit intelligent 100. En outre, le contrôleur 170 est configuré pour reconnaître différents utilisateurs utilisant le même lit intelligent 100 (par exemple, des membres de la famille). Par exemple, le contrôleur 170 utilise une entrée sonore (par exemple, à partir d'un microphone dans des capteurs environnementaux 180), ainsi que les données de poids précédentes et similaires pour différencier les utilisateurs.

Dans certains exemples, les capteurs BCG 310 sont utilisées pour surveiller la fréquence cardiaque (FC) de l'utilisateur, surveiller la fréquence respiratoire (FR) de l'utilisateur et / ou surveiller la fréquence de retournement au lit (RT). Par exemple, les capteurs BCG 310 comprennent des transducteurs polyvinylidènefluoride (PVDF).

Il convient de noter que la difficulté d'extraire les informations FC et / ou FR du signal BCG est principalement due à diverses interférences d'autres signaux de mouvement et de bruit avec les signaux FC et FR. En conséquence, les systèmes conventionnels qui sont limités à un type de capteurs BCG, tels que les transducteurs PVDF, les films électromécaniques (EMFi) ou les capteurs de force sont limités dans leur précision.

Contrairement aux systèmes conventionnels, le lit intelligent 100 permet de combiner l'entrée des capteurs BCG 310 et des capteurs de force 107. En outre, le module de surveillance de la santé 250 comprend un algorithme de filtrage adaptatif 255, dont diverses fonctions seront maintenant décrites plus en détail. Les capteurs BCG 310 sont utilisées pour détecter les signaux BCG bruts, tandis que les capteurs de force 107 sont utilisées pour détecter les mouvements de l'utilisateur, lors de sa présence sur le lit intelligent 100. Par exemple, la sensibilité des capteurs de force 107 est abaissée. L'algorithme de filtrage adaptatif 255 soustrait divers bruits, par exemple, associés aux mouvements de l'utilisateur dans le lit intelligent 100, les mouvements potentiels du lit intelligent 100, etc. (détectés par les capteurs de force 107) du signal BCG brut (obtenu à l'aide des capteurs BCG 310). Le signal BCG traité, produit par l'algorithme de filtrage adaptatif 255 est plus précis que le signal BCG brut des capteurs BCG 310.

En outre, une surveillance continue et prolongée de divers signaux BCG, décrite ci-dessus, est très avantageuse en comparaison avec des mesures peu fréquentes classiquement associées à ces types de signaux. Par exemple, cette surveillance continue permet d'améliorer le diagnostic et la surveillance de divers dysfonctionnements cardiaques et respiratoires, tels que la tachycardie, la bradycardie, l'apnée, la tachypnée et la bradypnée. Dans certains exemples, l'enregistrement des mouvements du corps par des capteurs de force 107 aide également au diagnostic et à la surveillance du syndrome des jambes sans repos (SJSR) puisque le module de surveillance de la santé 250 est configuré pour différencier diverses origines des mouvements du corps, par exemple des capteurs de force 107 positionnés plus près de la section des jambes 150 par rapport aux capteurs de force 107 positionnées plus près de la section de torse 140. Comme indiqué ci-dessus, ces analyses sont effectuées de manière continue et non invasive sans aucune action requise de l'utilisateur.

### Exemples de Gestion du Sommeil

En se référant à la Fig. 2, dans certains exemples, le contrôleur 170 comprend également le module de gestion du sommeil 260. Le module de gestion du sommeil 260 est configuré pour interagir et partager des données avec un ou plusieurs autres modules du contrôleur 170. Par exemple, le module de gestion du sommeil 260 est configuré pour recevoir les données FC, FR et RT du module de surveillance de la santé 250. Dans le même exemple ou dans d'autres exemples, le module de gestion du sommeil 260 est configuré pour recevoir le son, la température, l'humidité et d'autres données similaires du module d'environnement 210. Plus spécifiquement, le module de gestion du sommeil 260 est configuré pour identifier le ronflement à partir des données sonores, par exemple, obtenus par des microphones positionnés autour de la section de tête 130. Dans certains exemples, l'algorithme de détection du ronflement détermine si le son de ronflement coïncide avec l'inspiration (l'inhalation, au cours de laquelle le ronflement se produit) sur la base du signal de respiration, extrait du signal BCG. En tant que tel, la précision de la classification du ronflement est considérablement augmentée par rapport aux classificateurs sonores conventionnels. La détection du ronflement peut être importante pour le diagnostic de l'apnée obstructive du sommeil, qui amène une personne à arrêter temporairement de respirer pendant le sommeil et augmente le risque de diabète, d'obésité, d'hypertension, de crise cardiaque et d'autres problèmes cardiovasculaires.

Dans certains exemples, le module de gestion du sommeil 260 commande le fonctionnement d'un ou plusieurs entraînements, pour ajuster l'orientation d'une ou plusieurs sections suivantes : la section de tête 130, la section de torse 140 et / ou la section de jambes 150 par rapport au cadre respectif. En outre, dans certains exemples, le module de gestion du sommeil 260 est configuré pour envoyer des instructions au module de chauffage de lit 240, par exemple, pour ajuster la température d'une ou plusieurs couvertures chauffantes.

Dans certains exemples, le module de gestion du sommeil 260 comprend et utilise un algorithme de reconnaissance des phases du sommeil 265, qui identifie des étapes de sommeil particulières (par exemple, une étape d'éveil, une étape de sommeil léger, une étape de sommeil intermédiaire et une étape de sommeil profond) et autres paramètres associés (par exemple, la durée, la séquence, le taux de retournement, le départ du lit et les heures de début et de fin du sommeil). Le module de gestion du sommeil 260 utilise ces paramètres pour établir un score de sommeil. Par exemple, le sommeil parfait se voit attribuer un score maximum de 100%. Des points sont soustraits pour chaque événement ayant des effets négatifs sur la qualité du sommeil, tels que les retournements, la tachypnée, mettre trop de temps avant de s'endormir, aller au lit plus tard que l'heure recommandée. Par exemple, se lever plusieurs fois au cours peut faire baisser le score de sommeil de 10%, ce qui détermine le sommeil agité (p. Ex., se retourner excessivement au lit) -4%, se coucher plus tard que l'heure normale -8%, ne pas être capable de s'endormir pendant une période de temps prédéfinie -2%, détection de la tachypnée -2%. Dans l'exemple où toutes ces conditions sont présentes, la diminution totale est de 26%, ce qui ramène le score à 74%. Dans l'ensemble, chaque événement est associé à un «coefficient d'importance», qui détermine le nombre de points soustraits pour cet événement. Ces coefficients peuvent être adaptés à des utilisateurs spécifiques en fonction de divers facteurs associés à l'utilisateur, par exemple l'âge, le sexe, l'état de santé.

Dans certains exemples, le module de gestion du sommeil 260 génère un «Hypnogramme», qui est un profil horaire identifiant différents états de sommeil (par exemple, éveillé, sommeil léger, sommeil de niveau moyen et sommeil profond). D'une façon similaire, dans certains exemples, le module de gestion du sommeil 260 génère une courbe de score de sommeil, qui combine les scores de sommeil pour une période de temps définie (par exemple, une semaine, un mois, etc.). L'hypnogramme peut être présentée dans un rapport fourni à l'utilisateur. Dans certains exemples, le rapport indique également le pourcentage de temps correspondant à chaque état de veille.

Dans certains exemples, le score de sommeil est corrélé aux paramètres environnementaux, enregistrés au cours de la même période. Comme indiqué ci-dessus, certains exemples de ces paramètres comprennent la température, l'humidité, le niveau de bruit et le niveau de luminosité. Le module de gestion du sommeil 260 est configuré pour fournir des rapports et faire des recommandations d'ajustement d'un ou plusieurs paramètres environnementaux, par exemple, pour améliorer le score de sommeil / la qualité du sommeil. En outre, dans certains exemples, le module de gestion du sommeil 260 collecte des données contenant des scores de sommeil dans différentes conditions environnementales, compare les scores de sommeil à différents paramètres environnementaux, établit une corrélation entre la qualité du sommeil et les paramètres environnementaux, et recommande divers paramètres environnementaux pour améliorer le score de sommeil.

Dans certains exemples, le score de sommeil est également utilisé comme donnée de rétroaction pour ajuster divers paramètres du lit intelligent 100, tels que l'orientation d'une ou plusieurs section de tête 130, section de torse 140 et section des jambes 150 par rapport au cadre support y correspondant et / ou fermeté du matelas 120. Diverses caractéristiques pour les réglages de dureté sont décrites ci-dessous. D'autres exemples de paramètres qui peuvent être ajustés comprennent, mais sans s'y limiter, la lumière, le son, la température ambiante et l'humidité, produits par les ajusteurs d'environnement 190.

### Exemples de Fonctionnalités d'intimité

L'obtention d'informations à l'aide de divers capteurs environnementaux 180 soulève certains problèmes de confidentialité. Par exemple, les lits sont utilisés pour des moments intimes, et les gens peuvent ne pas vouloir que diverses données (audio, changements de poids, etc.) soient enregistrées et partagées à l'extérieur, par exemple à l'unité de service de lit intelligent 295. Pour répondre à ces problèmes de confidentialité, le lit intelligent 110 comprend diverses fonctionnalités à la fois au niveau matériel et au niveau logiciel. Par exemple, un microphone au niveau des capteurs environnementaux 180 est équipé d'un filtre spécial de perte d'informations, limitant les types de sons capturés par le microphone et transmis au contrôleur 170. Il convient de noter que la fonction de perte d'informations est implémentée dans les circuits électroniques des microphones.. Par exemple, les signaux de deux microphones sont fournis à un circuit analogique qui effectue des opérations mathématiques sur les signaux, avant que ce signal analogique ne soit converti en un signal numérique (par exemple, par un convertisseur analogique-numérique) et transmis au contrôleur 170. Le but des opérations mathématiques effectuées consistent à " perdre des informations " du signal qui sont importantes pour la reconnaissance par l'oreille humaine du contenu sonore mais qui ne sont pas importantes pour la tâche de classification sonore par le modèle d'IA qui peut être effectuée plus tard, par exemple, par le classificateur sonore 272 du contrôleur 170. En tant que tel, le signal audio est modifié avant même que le signal ne soit converti en un signal numérique, ce qui améliore la confidentialité et empêche la perte d'informations en cas de violation de la sécurité du système.

En outre, en se référant à la Fig. 2, dans certains exemples, le contrôleur 170 comprend le module d'intimité 270. Le module d'intimité 270, à son tour, comprend un modificateur de son 271, qui élimine tous les sons classés d'intimité du flux audio enregistré. Plus précisément, le modificateur de son 271 est configuré pour identifier ou au moins différencier les catégories suivantes: (1) une conversation normale, (2) une conversation rieuse et joyeuse, (3) des disputes et des cris, et (4) des sons liés à des activités sexuelles. Dans certains exemples, les mêmes opérations mathématiques, décrites ci-dessus dans le contexte des circuits analogiques, sont utilisées pour modifier les fichiers son dans l'apprentissage du modèle d'IA. En tant que tel, le modèle d'IA est capable de différencier et de classer les catégories de sons souhaitées.

Ces caractéristiques permettent au lit intelligent 100 de capturer l'audio à partir de l'environnement du lit sans collecter aucune information privée (telle que le contenu de la parole, les sons intimes, etc.). Le contrôleur 170 stocke et traite uniquement les catégories de classification énumérées ci-dessus. Tous les autres événements qui ne présentent aucun intérêt pour notre système ne sont ni captés ni compris.

Dans certains exemples, la sortie des capteurs de force 107 est également utilisée comme source d'informations sur des actes intimes potentiels. Le profil de force (dans le temps) enregistré par les capteurs de force 107 correspondant aux actes intimes est distinct des autres activités du lit intelligent 100. Cette sortie peut être utilisée pour compléter la détermination de l'une des quatre catégories énumérées ci-dessus par le modificateur de son 271. En d'autres termes, le modificateur de son 271 utilise une combinaison de sons et de signaux de force pour déterminer la classification correcte. Par exemple, la sortie reçue des capteurs de force 107 peut être utilisée pour déterminer l'intensité, la durée et le temps de diverses activités.

En outre, les informations des capteurs de force 107 fournissent des informations sur le fait que le couple a commencé à séparer ses couchages, ce qui peut être utilisé comme indicateur d'une évolution négative de la relation conjugale.

Dans certains exemples, le module d'intimité 270 est utilisé comme moyen de communication entre les partenaires en configurant le diffuseur d'arôme, la musique et l'éclairage de la pièce sur une configuration particulière qui peut signaler un message donné à l'autre partenaire. Cela peut même être réglé automatiquement si le couple choisit de laisser le système les surprendre avec ses propres actions lorsqu'il remarque que le score de la relation a besoin d'être amélioré ou s'il remarque - via le système de sonorisation - que l'ambiance est adéquate pour s'amuser. Dans certains exemples, le module d'intimité 270 est configuré pour contrôler un diffuseur d'arôme, fourni dans les ajusteurs d'environnement 190. Par exemple, le module d'intimité 270 peut demander au diffuseur d'arôme d'infuser un parfum autour du lit intelligent 100, lors de la détection d'activités intimes dans le lit intelligent 100 Dans certains exemples, le module d'intimité 270 est configuré pour contrôler un haut-parleur, fourni dans les ajusteurs d'environnement 190. Par exemple, le module d'intimité 270 peut ordonner au haut-parleur de jouer des sons sélectionnés (par exemple, mélodie, chanson, etc.), lors de la détection d'activités dans le lit intelligent 100. Dans certains exemples, le module d'intimité 270 est configuré pour contrôler une lumière, fournie dans les ajusteurs d'environnement 190. Par exemple, le module d'intimité 270 peut demander à la lumière (par exemple, une bande LED) de changer d'intensité (par exemple, diminué), de couleur, etc. lors de la détection d'activités intimes dans le lit intelligent 100.

Dans certains exemples, le module d'intimité 270 est configuré pour donner une note à une relation intime basée sur au moins le son déformé. Plus spécifiquement, une autre entrée de capteur (par exemple, les capteurs de force) est utilisée. Par exemple, le score peut être établi au fil du temps en se basant sur la fréquence des rapports sexuels, la fréquence des couchages séparés, le pourcentage de temps passé à s'amuser au lit par rapport au temps passé à se disputer et à crier, le pourcentage de temps passé à dormir en étreinte ou en position très proche par rapport au fait de dormir dans le même lit mais à distance (par exemple, en utilisant les capteurs de force) et des informations sur la position de chaque personne dans le lit. Dans certains exemples, le score et la tendance sont partagés avec les utilisateurs du lit intelligent 100 ou même partagés avec le thérapeute du couple (par exemple, sur la base d'une autorisation spécifique des utilisateurs). Dans l'ensemble, le module d'intimité 270 est configuré pour fournir une preuve objective de l'évolution de la relation, en comparaison avec les récits subjectifs fournis conventionnellement par chaque partie au cours d'une séance de thérapie.

### Exemples de Fonctionnement de Lits Intelligents Multifonctionnels

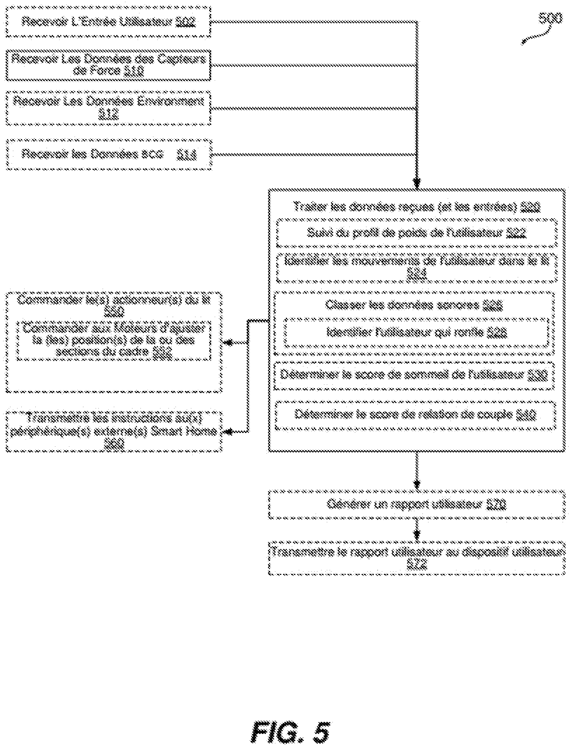

La Fig. 5 est un organigramme de processus correspondant au procédé 500 de fonctionnement du lit intelligent 100, conformément à quelques exemples.

Le procédé 500 peut impliquer la réception d'une entrée utilisateur (bloc 502). L'utilisateur peut fournir une entrée via l'interface utilisateur 280 du contrôleur 170 et / ou le dispositif utilisateur 290, qui est couplé en communication au lit intelligent 100. Certains exemples de l'entrée utilisateur comprennent, par exemple, la dureté du matelas, la sélection du son, la sélection de la lumière, la température de la couverture chauffante, etc.

Le procédé 500 peut impliquer la réception de données des capteurs de force (bloc 510). Les données des capteurs de force représentent le poids à chaque pied du lit intelligent 100. Ces données peuvent être recueillies en continu et sont utilisées, par exemple, pour déterminer quand l'utilisateur est entré dans le lit, a quitté le lit, se retourne dans le lit, pour surveiller le poids de l'utilisateur, etc.

Le procédé 500 peut impliquer la réception de données environnementales (bloc 512). Divers exemples de données environnementales sont décrits ci-dessous, par exemple la température ambiante, la lumière, le bruit, l'humidité, la qualité de l'air. Ces données sont utilisées par le contrôleur 170, par exemple, pour déterminer l'effet de l'environnement sur la qualité du sommeil.

Le procédé 500 peut impliquer la réception de données BCG (bloc 514). Par exemple, les données BCG peuvent être reçues du capteur BCG 310, installée dans le matelas 120 comme, par exemple, décrit ci-dessus en référence à la Fig. 3.

Le procédé 500 peut impliquer la réception et le traitement des entrées et des données reçues (bloc 520). Différents types de traitement de données entrent dans le champ d'application, tels que le suivi du profil de poids de l'utilisateur (bloc 522), l'identification des mouvements de l'utilisateur au lit (bloc 524), la classification des données sonores (bloc 526) de manière à identifier le ronflement (bloc 528), et déterminer le score de sommeil de l'utilisateur (bloc 530) et le score de la relation de couple (bloc 540).

Le procédé 500 peut impliquer l'instruction d'un ou plusieurs actionneurs de lit (bloc 550) pour exécuter diverses fonctions, telles que le réglage des positions d'une ou plusieurs sections du cadre (bloc 552).

Le procédé 500 peut impliquer la transmission d'instructions à des dispositifs de maison intelligente externes (bloc 560). Par exemple, le contrôleur 170 peut envoyer des instructions à un thermostat intelligent pour ajuster la température de l'environnement autour du lit.

Le procédé 500 peut impliquer la génération d'un rapport d'utilisateur (bloc 570) et la transmission de ce rapport aux dispositifs d'utilisateur (bloc 572).

La Fig. 6 est un organigramme de processus correspondant à la méthode 600 pour l'identification de l'utilisateur et la classification sonore. La méthode 600 utilise un son modifié / déformé comme entrée, pour assurer la confidentialité, et est reçue (bloc 610) du lit intelligent 100. En tant que telle, la méthode 600 peut être exécutée par un système, qui est distant mais connecté au lit intelligent 100. Dans certains exemples, le procédé 600 comprend la détermination (bloc de décision 620) si le son modifié est un son lié à l'homme, par exemple pour différencier le son modifié du silence ou d'autres sons non humains (par exemple, les sons de la rue, les sons du système de ventilation, etc.). Une fois que le son modifié est identifié comme humain, le procédé 600 procède à la détermination (bloc 630) si le son modifié correspond à un ou plusieurs locuteurs. S'il y a plusieurs locuteurs (bloc de décision 640), alors le procédé 600 procède à la réalisation (bloc 650) de la séparation de la voix des différents locuteurs individuels. Le procédé 600 procède ensuite à la reconnaissance vocale (bloc 660) pour attribuer différentes parties audio à chaque locuteur. La méthode 600 procède ensuite à une analyse des sentiments (bloc 670) où le son modifié (séparé pour chaque locuteur individuel) est classé dans une ou plusieurs des catégories suivantes: ronflement, conversation normale, conversation joyeuse, cris, conversation tendue et sons intimes . Dans certains exemples, la reconnaissance du locuteur est mise en oeuvre en demandant à un utilisateur de prononcer une phrase donnée dans le microphone de son téléphone intelligent ou en utilisant des microphones dans le lit intelligent 110. Un modèle d'IA entraîné extrait une empreinte vocale de cet audio et l'utilise pour reconnaître la voix de l'utilisateur. Dans le cas d'un lit de deux personnes, deux audio sont collectés.

### Exemples de Systèmes Informatiques

La Fig. 7 est un schéma de principe correspondant au système informatique 700 et au programme informatique 722, qui sont utilisés pour supporter et mettre en oeuvre diverses fonctions du lit intelligent 100 décrites ci-dessus. Spécifiquement, divers composants du lit intelligent 100 (par exemple, le contrôleur 170) peuvent être mis en oeuvre et pris en charge en tant que composants du système informatique 700 et du programme informatique 722, par exemple des modules matériels et / ou logiciels. Dans certains exemples, le système informatique 700 comprend le bus 702, qui assure les communications entre le processeur 704 et divers modules. Le processeur 704 est configuré pour exécuter des instructions pour un logiciel (par exemple, le programme informatique 722).

Divers processus du lit intelligent 100 sont exécutés par le processeur 704 en utilisant des instructions implémentées par ordinateur. Ces instructions sont appelées code programme 718, code programme utilisable par ordinateur ou code programme lisible par ordinateur qui est lu et exécuté par un processeur dans le processeur 704. Le code programme dans différents exemples est incorporé sur différents supports de stockage physiques ou lisibles par ordinateur.

Le code programme 718 est stocké sous une forme fonctionnelle sur un support lisible par ordinateur 720 qui est sélectivement amovible et est chargé sur ou transféré vers le système informatique 700 pour exécution par le processeur 704. Dans ces exemples illustratifs, Le code programme 718 et le support lisible par ordinateur 720 forment le programme informatique 722. Dans un exemple, le support lisible par ordinateur 720 est ou comprend un support de stockage lisible par ordinateur 724. Dans ces exemples illustratifs, le support de stockage lisible par ordinateur 724 est un dispositif de stockage physique ou tangible utilisé pour stocker le code programme 718 plutôt qu'un support qui propage ou transmet le code programme 718.

Le système informatique 700 comprend un ou plusieurs modules, conçus pour mettre en oeuvre diverses fonctions du lit intelligent 100. Par exemple, le système informatique 700 comprend un module de commande de capteurs environnementaux 730, qui reçoit et traite (par exemple, prétraiter / traiter partiellement) l'entrée provenant des capteurs environnementaux 180. Dans certains exemples, le système informatique 700 comprend un module de commande de capteurs de force 732, qui reçoit et traite (par exemple, prétraitement / traitement partiel) l'entrée des capteurs de force 107. Dans certains exemples, le système informatique 700 comprend un module de commande de capteur BCG 734, qui reçoit et traite (par exemple, prétraitement / traitement partiel) une entrée du capteur BCG 310. Dans certains exemples, le système informatique 700 comprend le module de commande de moteur 736, qui fournit une entrée à l'entraînement 161, l'entraînement 162 et / ou l'entraînement 163. En outre, le module de commande de moteur 736 peut recevoir diverses entrées, telles que la position de chaque section du lit intelligent 100. Dans certains exemples, le système informatique 700 comprend un module de commande de couverture chauffante 738, qui fournit une entrée à la couverture chauffante. En outre, le module de commande de couverture chauffante 738 peut recevoir diverses entrées, telles que la température de l'environnement, la température de la couverture, la température des unités en mousse viscoélastique 410 dans le matelas. Dans certains exemples, le système informatique 700 comprend le module de commande d'éclairage 740, qui fournit une entrée aux lumières ou, plus généralement, aux ajusteurs d'environnement 190. Dans certains exemples, le système informatique 700 comprend également le module de commande de fermeté du matelas 742, qui fournit une entrée aux unités de chauffage 420 du matelas 120. Le système informatique 700 comprend également l'unité de communication 710, qui fournit des communications avec d'autres systèmes ou dispositifs informatiques, tels que les dispositifs d'utilisateur 290 et / ou l'unité de service du lit intelligent 295. Par exemple, l'unité de communication 710 est une carte d'interface réseau, Bluetooth module, etc.

### Conclusion

Bien que les concepts précédents aient été décrits de manière assez détaillée à des fins de clarté de compréhension, il sera évident que certains changements et modifications peuvent être mis en pratique dans le cadre des revendications annexées. Il convient de noter qu'il existe de nombreuses autres manières de mettre en oeuvre les processus, les systèmes et les appareils. En conséquence, les présents exemples doivent être considérés comme illustratifs et non restrictifs.

## Revendications

1. Lit intelligent multifonctionnel (100) comprenant:
- un cadre (110) comprenant de multiples sections, mobiles indépendamment les unes par rapport aux autres;
- une pluralité de pieds (106) comprenant chacun un capteur de force, couplé au cadre;
- un matelas (120) positionné sur et supporté par le cadre;
- une pluralité de capteurs environnementaux (180) comprenant un ou plusieurs microphones, configurés pour obtenir un son réel autour du lit intelligent multifonctionnel, et pour déformer le son réel, générant ainsi de manière irréversible un son déformé, non reconnaissable pour un être humain, à l'aide d'une fonction mathématique irréversible, implémentée dans les circuits électroniques des microphones par logiciel ou par matériel, dans lequel le son déformé comprend des caractéristiques distinctives permettant au classificateur d'intelligence artificielle (IA) de différencier les différentes catégories de sons;
- un contrôleur (170) couplé en communication et configuré pour recevoir et traiter l'entrée des capteurs de force dans chacun de la pluralité des pieds et de chacun de la pluralité des capteurs environnementaux; et
- une pluralité d'actionneurs (160), couplés en communication au contrôleur, couplés mécaniquement au cadre, et configurés pour changer au moins une ou plusieurs caractéristiques du lit intelligent multifonctionnel en fonction d'entrées provenant du contrôleur.

2. Lit intelligent multifonctionnel selon la revendication 1, dans lequel la ou les caractéristiques du lit comprennent au moins une parmi les suivantes : le contrôle de la fermeté du matelas ou les positions des multiples sections du cadre l'une par rapport à l'autre.

3. Lit intelligent multifonctionnel selon la revendication 1, dans lequel le contrôleur est configuré pour déterminer la présence d'un ou plusieurs utilisateurs dans le lit intelligent multifonctionnel, sur la base de l'entrée des capteurs de force dans chacune de la pluralité des pieds du lit et pour déterminer l'identité de un ou de plusieurs utilisateurs dans le lit intelligent multifonctionnel.

4. Lit intelligent multifonctionnel selon la revendication 3, dans lequel le contrôleur comprend un classificateur sonore, configuré pour déterminer l'identité du ou des utilisateurs dans le lit intelligent multifonctionnel.

5. Lit intelligent multifonctionnel selon la revendication 3, comprenant en outre un capteur de balistocardiographie, couplé en communication au contrôleur et configuré pour déterminer au moins l'une des fréquences suivantes : une fréquence cardiaque, une fréquence respiratoire ou une fréquence de retournement du ou des utilisateurs dans le lit intelligent multifonctionnel et dans lequel le contrôleur est configuré pour faire correspondre au moins l'un de la fréquence cardiaque, de la fréquence respiratoire ou de la fréquence de retournement à l'identité de un ou plusieurs utilisateurs dans le lit intelligent multifonctionnel.

6. Lit intelligent multifonctionnel selon la revendication 1, dans lequel la pluralité de capteurs environnementaux comprend de multiples microphones, configurés pour différencier le ronflement de multiples utilisateurs du lit intelligent multifonctionnel.

7. Lit intelligent multifonctionnel selon la revendication 1, dans lequel le contrôleur reçoit le son déformé du ou des microphones et n'a pas accès au son réel.

8. Lit intelligent multifonctionnel selon la revendication 1, dans lequel le contrôleur est configuré pour évaluer une relation intime sur la base d'au moins le son déformé.

9. Lit intelligent multifonctionnel selon la revendication 1, dans lequel le contrôleur est configuré pour identifier une ou plusieurs catégories de sons dans le son déformé, la ou les catégories de sons sont sélectionnées dans le groupe consistant en une conversation normale, une conversation riante et joyeuse, des discussions et des cris ainsi que des actes intimes.

10. Lit intelligent multifonctionnel selon la revendication 9, dans lequel le contrôleur est configuré pour analyser collectivement la ou les catégories de sons identifiés ensemble avec les entrées depuis les capteurs de force dans chacun de la pluralité de pieds de lit.

11. Procédé de fonctionnement d'un lit intelligent multifonctionnel selon la revendication 1, le procédé comprenant:
Recevoir des données des capteurs de force dans chacun d'une pluralité de pieds du lit intelligent multifonctionnel, la pluralité de pieds supportant un cadre du lit intelligent multifonctionnel; Traiter les données, reçues des capteurs de force, en utilisant un contrôleur du lit intelligent multifonctionnel; et Ajuster au moins une ou plusieurs caractéristiques du lit intelligent multifonctionnel à l'aide d'une pluralité d'actionneurs du lit intelligent multifonctionnel, sur la base d'une entrée du contrôleur.
- l'obtention d'un son réel autour du lit intelligent multifonctionnel en utilisant un ou plusieurs microphones du lit intelligent multifonctionnel
- utiliser le ou les microphones, déformer le son réel, générant ainsi de manière irréversible un son déformé à l'aide d'une fonction mathématique irréversible, implémentée dans un logiciel ou un matériel,
dans lequel le son déformé comprend des caractéristiques distinctives pour différencier différentes catégories de sons,
- fournir le son déformé à un contrôleur du lit intelligent multifonctionnel.

12. Procédé de fonctionnement d'un lit intelligent multifonctionnel selon la revendication 11, le procédé comprenant :
- l'obtention de données de ballistocardiogramme à partir d'un capteur de balistocardiograhie du lit intelligent multifonctionnel, dans lequel les données, reçues des capteurs de force, sont traitées par le contrôleur conjointement avec les données du ballistocardiogramme pour générer l'entrée pour ajuster au moins une ou plusieurs caractéristiques du lit intelligent multifonctionnel.

13. Procédé de fonctionnement d'un lit intelligent multifonctionnel selon la revendication 11, le procédé comprenant :
- l'identification, au niveau du contrôleur, d'une ou plusieurs des catégories de sons dans le son déformé, les catégories de sons sont sélectionnées dans le groupe constitué d'une conversation normale, d'une conversation riante et joyeuse, d'une dispute , de cris ainsi que des actes intimes.

14. Procédé de fonctionnement d'un lit intelligent multifonctionnel selon la revendication 11, le procédé comprenant :
- l'analyse du son déformé, au niveau du contrôleur, pour obtenir au moins une fonction parmi les suivantes : la séparation des sources vocales de plusieurs locuteurs, la reconnaissance vocale ou l' analyse des sentiments.

## Patentansprüche

1. Multifunktionales intelligentes Bett (100), welches umfasst:
- einen Rahmen (110), der mehrere Abschnitte umfasst, die unabhängig voneinander beweglich sind;
- mehrere Füße (106), die jeweils einen Kraftsensor umfassen, der mit dem Rahmen gekoppelt ist;
- eine Matratze (120), die auf dem Rahmen angeordnet ist und von diesem getragen wird;
- mehrere Umgebungssensoren (180), die ein oder mehrere Mikrofone umfassen,
die ausgestaltet sind, einen realen Ton um das multifunktionale intelligente Bett herum zu erhalten und den realen Ton zu verzerren, wodurch irreversibel ein verzerrter Ton erzeugt wird, der für einen Menschen nicht erkennbar ist, mit Hilfe einer irreversiblen mathematischen Funktion, die in den elektronischen Schaltungen der Mikrofone durch Software oder Hardware implementiert ist, worin der verzerrte Ton Unterscheidungsmerkmale umfasst, die es dem Klassifikator für künstliche Intelligenz (IA) ermöglichen, zwischen verschiedenen Tonkategorien zu unterscheiden;
- eine Steuerung (170), die kommunikativ gekoppelt und ausgestaltet ist, die Eingabe der Kraftsensoren in jedem der mehreren Füße und von jedem der mehreren Umgebungssensoren zu empfangen und zu verarbeiten; und
- mehrere Aktuatoren (160), die kommunikativ mit der Steuerung gekoppelt sind, mechanisch mit dem Rahmen gekoppelt und ausgestaltet sind, mindestens eine oder mehrere Merkmale des multifunktionalen intelligenten Bettes auf der Grundlage von Eingaben von der Steuerung zu ändern.

2. Multifunktionales intelligentes Bett nach Anspruch 1, worin das oder die Merkmale des Bettes mindestens eines umfasst von: die Steuerung der Festigkeit der Matratze oder die Positionen der mehreren Abschnitte des Rahmens in Bezug zueinander.

3. Multifunktionales intelligentes Bett nach Anspruch 1, worin die Steuerung ausgestaltet ist, dass sie die Anwesenheit eines oder mehrerer Benutzer in dem multifunktionalen intelligenten Bett auf der Grundlage der Eingabe der Kraftsensoren in jedes der mehreren Beine des Bettes bestimmt und die Identität eines oder mehrerer Benutzer in dem multifunktionalen intelligenten Bett bestimmt.

4. Multifunktionales intelligentes Bett nach Anspruch 3, worin die Steuerung einen Ton-Klassifikator umfasst, der ausgestaltet ist, die Identität des oder der Benutzer in dem multifunktionalen intelligenten Bett zu bestimmen.

5. Multifunktionales intelligentes Bett nach Anspruch 3, welches ferner einen Balistokardiographie-Sensor umfasst, der mit der Steuereinheit kommunikativ gekoppelt und ausgestaltet ist, mindestens eine der folgenden Frequenzen zu bestimmen: eine Herzfrequenz, eine Atemfrequenz oder eine Umdrehungsfrequenz des oder der Benutzer in dem multifunktionalen intelligenten Bett, und worin die Steuereinheit ausgestaltet ist, mindestens eine der Herzfrequenz, der Atemfrequenz oder der Umdrehungsfrequenz mit der Identität eines oder mehrerer Benutzer in dem multifunktionalen intelligenten Bett in Übereinstimmung zu bringen.

6. Multifunktionales intelligentes Bett nach Anspruch 1, worin die mehreren Umgebungssensoren mehrere Mikrofone umfassen, die ausgestaltet sind, das Schnarchen mehrerer Benutzer des multifunktionalen intelligenten Bettes zu unterscheiden.

7. Multifunktionales intelligentes Bett nach Anspruch 1, worin die Steuerung den verzerrten Ton des Mikrofons oder der Mikrofone empfängt und keinen Zugriff auf den realen Ton hat.

8. Multifunktionales intelligentes Bett nach Anspruch 1, worin die Steuerung ausgestaltet ist, eine intime Beziehung auf der Grundlage von mindestens dem verzerrten Ton zu bewerten.

9. Multifunktionales intelligentes Bett nach Anspruch 1, worin die Steuerung ausgestaltet ist, eine oder mehrere Tonkategorien in dem verzerrten Ton zu identifizieren, worin die eine oder die mehreren Tonkategorien ausgewählt sind, aus der Gruppe bestehend aus normaler Konversation, lachender und fröhlicher Konversation, Diskussionen und Schreien sowie intimen Handlungen.

10. Multifunktionales intelligentes Bett nach Anspruch 9, worin die Steuerung ausgestaltet ist, die eine oder die mehreren identifizierten Tonkategorien zusammen mit den Eingaben von den Kraftsensoren in jedem der mehreren Bettbeine kollektiv zu analysieren.

11. Verfahren zum Betreiben eines multifunktionalen intelligenten Bettes nach Anspruch 1,
wobei das Verfahren umfasst:
- Empfangen von Daten von den Kraftsensoren in jedem von mehreren Beinen des multifunktionalen intelligenten Bettes, worin die mehreren Beine einen Rahmen des multifunktionalen intelligenten Bettes tragen;
- Verarbeiten der von den Kraftsensoren empfangenen Daten unter Verwendung einer Steuerung des multifunktionalen intelligenten Bettes; und
- Einstellen von mindestens einem oder mehreren Merkmalen des multifunktionalen intelligenten Bettes mit Hilfe mehrerer Aktuatoren des multifunktionalen intelligenten Bettes auf der Grundlage einer Eingabe von der Steuerung;
- Erzeugung eines realen Tons um das multifunktionale intelligente Bett herum unter Verwendung eines oder mehrerer Mikrofone des multifunktionalen intelligenten Bettes;
- Verwenden des Mikrofons oder der Mikrofone, Verzerren des realen Tons, wodurch irreversibel ein verzerrter Ton mit Hilfe einer irreversiblen mathematischen Funktion erzeugt wird, die in einer Software oder Hardware implementiert ist,
worin der verzerrte Ton Unterscheidungsmerkmale zur Unterscheidung verschiedener Tonkategorien enthält,
- Bereitstellen des verzerrten Tons an eine multifunktionale intelligente Bettsteuerung.

12. Verfahren zum Betreiben eines multifunktionalen intelligenten Bettes nach Anspruch 11, worin das Verfahren umfasst:
- Erhalten von Ballistokardiogrammdaten von einem Ballistokardiogrammsensor des multifunktionalen intelligenten Bettes, worin die von den Kraftsensoren empfangenen Daten von der Steuereinheit zusammen mit den Ballistokardiogrammdaten verarbeitet werden, um die Eingabe zum Einstellen von mindestens einer oder mehreren Eigenschaften des multifunktionalen intelligenten Bettes zu erzeugen.

13. Verfahren zum Betreiben eines multifunktionalen intelligenten Bettes nach Anspruch 11, wobei das Verfahren umfasst:
- Identifizieren einer oder mehrerer der Tonkategorien in dem verzerrten Ton in der Steuerung, worin die Tonkategorien ausgewählt sind, aus der Gruppe bestehend aus einem normalen Gespräch, einem lachenden und fröhlichen Gespräch, einem Streit, Schreien und intimen Handlungen.

14. Verfahren zum Betreiben eines multifunktionalen intelligenten Bettes nach Anspruch 11, wobei das Verfahren umfasst:
- Analyse des verzerrten Tons in der Steuerung, um mindestens eine der folgenden Funktionen zu erreichen: Trennen der Sprachquellen von mehreren Sprechern, Spracherkennung oder Gefühlsanalyse.

## Claims

1. Multifunctional smart bed (100) comprising:
- a frame (110) comprising a plurality of sections that are movable independently of each other;
- a plurality of feet (106), each comprising a force sensor coupled to the frame;
- a mattress (120) arranged on the frame and supported by it;
- a plurality of environmental sensors (180) comprising one or more microphones,
designed to obtain a real sound around the multifunctional intelligent bed and to distort the real sound, thereby irreversibly generating a distorted sound that is not recognisable to a human, by means of an irreversible mathematical function implemented in the electronic circuits of the microphones by software or hardware, wherein the distorted sound comprises distinguishing features that enable the artificial intelligence (IA) classifier to distinguish between different sound categories;
- a controller (170) communicatively coupled and configured to receive and process input from the force sensors in each of the plurality of feet and from each of the plurality of environmental sensors; and
- a plurality of actuators (160) communicatively coupled to the controller, mechanically coupled to the frame, and configured to change at least one or more features of the multifunctional smart bed based on inputs from the controller.

2. The multifunctional smart bed according to claim 1, wherein the feature or features of the bed comprises at least one of: controlling the firmness of the mattress or the positions of the multiple sections of the frame in relation to each other.

3. The multifunctional smart bed of claim 1, wherein the controller is configured to determine the presence of one or more users in the multifunctional smart bed based on input from the force sensors in each of the plurality of legs of the bed and to determine the identity of one or more users in the multifunctional smart bed.

4. The multifunctional smart bed according to claim 3, wherein the controller comprises a sound classifier adapted to determine the identity of the user or users in the multifunctional smart bed.

5. The multifunctional smart bed of claim 3, further comprising a balistocardiography sensor communicatively coupled to the control unit and configured to determine at least one of a heart rate, a respiratory rate, or a rotation rate of the user or users in the multifunctional smart bed, and wherein the control unit is configured to match at least one of the heart rate, the respiratory rate, or the rotation rate to the identity of one or more users in the multifunctional smart bed.

6. The multifunctional smart bed of claim 1, wherein the plurality of environmental sensors comprise a plurality of microphones configured to distinguish snoring of a plurality of users of the multifunctional smart bed.

7. The multifunctional smart bed according to claim 1, wherein the controller receives the distorted sound of the microphone or microphones and has no access to the real sound.

8. The multifunctional smart bed according to claim 1, wherein the controller is adapted to evaluate an intimate relationship based on at least the distorted sound.

9. The multifunctional smart bed according to claim 1, wherein the controller is adapted to identify one or more sound categories in the distorted sound, wherein the one or more sound categories are selected from the group consisting of normal conversation, laughing and cheerful conversation, discussions and shouting, and intimate actions.

10. The multifunctional smart bed of claim 9, wherein the controller is adapted to collectively analyse the one or more identified sound categories together with the inputs from the force sensors in each of the plurality of bed legs.

11. Method of operating a multifunctional smart bed according to claim 1,
wherein the method comprises:
- receiving data from the force sensors in each of a plurality of legs of the multifunctional smart bed, wherein the plurality of legs support a frame of the multifunctional smart bed;
- processing the data received from the force sensors using a controller of the multifunctional smart bed; and
- adjusting at least one or more features of the multifunctional smart bed using a plurality of actuators of the multifunctional smart bed based on an input from the controller;
- generating a real sound around the multifunctional smart bed using one or more microphones of the multifunctional smart bed;
- using the microphone or microphones, distorting the real sound, irreversibly creating a distorted sound using an irreversible mathematical function implemented in software or hardware,
in which the distorted sound contains distinguishing features to differentiate between different sound categories,
- providing the distorted sound to a multifunctional intelligent bed control.

12. The method of operating a multifunctional smart bed according to claim 11, wherein the method comprises:
- receiving ballistocardiogram data from a ballistocardiogram sensor of the multifunctional smart bed, wherein the data received from the force sensors is processed by the control unit together with the ballistocardiogram data to generate the input for adjusting at least one or more characteristics of the multifunctional smart bed.

13. The method of operating a multifunctional smart bed according to claim 11, wherein the method comprises:
- identifying one or more of the sound categories in the distorted sound in the control in which the sound categories are selected from the group consisting of a normal conversation, a laughing and cheerful conversation, an argument, shouting and intimate actions.

14. The method of operating a multifunctional smart bed according to claim 11, wherein the method comprises:
- analysing the distorted sound in the control unit to achieve at least one of the following functions: Separating speech sources from multiple speakers, speech recognition or emotion analysis.
